(19) Europäisches Patentamt
European Patent Office
Office européen des brevets

(11) Numéro de publication: 0 015 817 A1

(12) **DEMANDE DE BREVET EUROPEEN**

(21) Numéro de dépôt: 80400258.2

(22) Date de dépôt: 25.02.80

(51) Int. Cl.³: C 07 D 211/46, A 61 K 31/445

(30) Priorité: 09.03.79 FR 7906070

(43) Date de publication de la demande: 17.09.80 Bulletin 80/19

(84) Etats contractants désignés: DE NL SE

(71) Demandeur: Société dite : METABIO-JOULLIE, 23-25 Avenue Morane-Saulnier, F-92360 Meudon La Foret (FR)

(72) Inventeur: Warolin, Christian, 55 Boulevard de Beauséjour, F-75016 Paris (FR)
Inventeur: Schneider, Roland, 88 Rue Pasteur, F-78700 Conflans Sainte Honorine (FR)

(74) Mandataire: Bressand, Georges et al, c/o CABINET LAVOIX 2 Place d'Estienne d'Orves, F-75441 Paris Cedex 09 (FR)

(54) **Nouveaux dérivés de la pipéridine, leur procédé de préparation et leur application en thérapeutique.**

(57) L'invention a pour objet la benzyloxy-4-pipéridine et ses sels pharmaceutiquement acceptables.

Ces composés sont utiles en thérapeutique comme antispasmodiques.

EP 0 015 817 A1

# Nouveaux dérivés de la pipéridine, leur procédé de préparation et leur application en thérapeutique.

La présente invention concerne de nouveaux dérivés de la pipéridine, leur procédé de préparation et leur application en thérapeutique.

La présente invention a pour objet la benzyloxy-4 pipéridine de formule

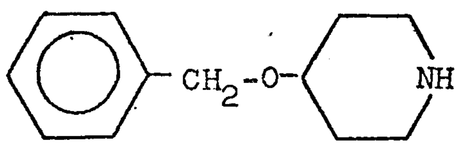

(I)

et ses sels d'addition avec des acides pharmaceutiquement acceptables.

La benzyloxy-4-pipéridine et ses sels peuvent être préparés de la façon suivante :

a) on condense l'hydroxy-4 pipéridine avec le chlorure de p-nitrobenzoyle en présence d'un accepteur d'acide, de façon à obtenir une pipéridine protégée de formule

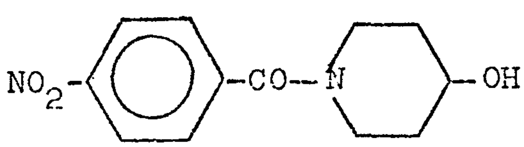

(II)

b) on effectue une benzylation du composé de formule II au moyen de chlorure de benzyle en présence d'un agent de condensation et d'un accepteur d'acide de façon à obtenir un composé de formule

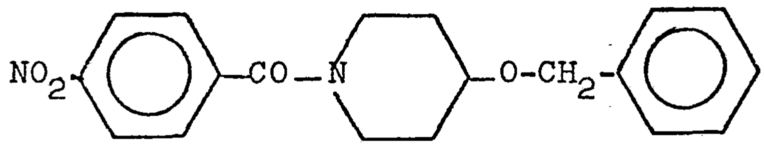 (III)

c) on libère le groupe aminé de la pipéridine, et

d) éventuellement on salifie la base ainsi obtenue.

L'exemple suivant illustre la préparation du composé de formule I.

EXEMPLE

a) p-nitrobenzoyl-1 hydroxy-4 pipéridine

Dans un erlenmeyer de 1 l, muni d'une agitation magnétique et disposé dans un bain d'eau, une solution de 30,34 g (0,3 mole) d'hydroxy-4 pipéridine et de 58,04 g de carbonate de potassium anhydre (0,42 mole) dans 260 ml d'eau est additionnée de 260 ml de chloroforme. Le milieu agité est additionné de 48,43 g (0,261 mole) de chlorure de p-nitrobenzoyle, par fractions.

Le milieu hétérogène est agité 30 mn à température ambiante, puis il est dilué par adjonction de 260 ml d'éther. Après 15 mn d'agitation, le précipité est essoré sous faible dépression, puis il est lavé à l'eau par 5 x 50 ml, jusqu'à absence d'ions $Cl^-$.

Le produit jaune pâle est séché sous vide 2 à 10 heures, en chauffant à 40-50°C.

On obtient 52,39 g de produit (Rdt 80 %)

F = 205-206°C (banc Kofler)

b) p-nitrobenzoyl-1 benzyloxy-4 pipéridine

Sous la hotte, à une suspension bien agitée de 33,21 g (0,133 mole) de p-nitrobenzoyl-1 hydroxy-4 pipéridine dans 176 ml de chlorure de benzyle est additionnée une solution de 53,2 g de soude à 50 % en poids (0,665 mole) puis, par fractions car la réaction est exothermique, 2,25 g de TBAS (sulfate acide de tétrabutylammonium), sont ajoutés.

Le milieu bien agité est porté 4 heures à 50°C, puis il est dilué par 345 ml d'eau pour dissoudre le NaCl ayant précipité, puis par 210 ml de chloroforme.

Le milieu est décanté, la phase aqueuse est extraite par 100 ml de $CHCl_3$ et la phase organique totale est lavée par 3 x 100 ml d'eau et séchée sur $MgSO_4$ une nuit. Après filtration, le chloroforme est chassé sous vide en évaporateur rotatif, et la solution résiduelle de produit dans le chlorure de benzyle est versée lentement, sous bonne agitation, dans 1760 ml de cyclohexane.

Le précipité formé, jaune très pâle, est agité environ 3 heures dans le milieu, puis il est filtré et lavé au cyclohexane et séché sous vide à 40°C.

On obtient 39,24 g de produit (Rdt 86,5%)

F = 119°C (Kofler).

c) Bromhydrate de benzyloxy-4 pipéridine

Une suspension agitée de 22,12 g (0,065 mole) de p-nitrobenzoyl-1 benzyloxy-4 pipéridine dans 390 ml d'éthanol à 96° est additionnée de 97,5 ml de potasse aqueuse 10 N (0,975 mole). Le milieu est agité la nuit à température ambiante, puis le lendemain, 4 heures à 50°C, sous azote.

L'éthanol est chassé sous vide au rotavapor (bain 35-40°C). Le résidu est redissous dans

160 ml d'eau, 60 g de NaCl pur sont ajoutés pour relarguer le produit qui est extrait par 160 ml d'éther.

Le mélange de NaCl et de sel de sodium de l'acide p-nitrobenzoïque est filtré, le filtrat est décanté, la phase éthérée est séchée une nuit sur $MgSO_4$, après extraction de la phase aqueuse par 3 x 160 ml d'éther.

Le solvant est séché sous vide, sans chauffer : de longues aiguilles jaune pâle se forment, à partir de l'huile initiale.

On obtient 12,28 g de produit (98,8 %).

La base est très hygroscopique.

Cette huile est dissoute dans 660 ml d'éther sec et dépéroxydé, puis additionnée peu à peu, en refroidissant dans un bain de glace, de 55 ml d'une solution environ 1,3 M d'acide bromhydrique gazeux dans de l'éther dépéroxydé sec (une solution plus concentrée, 4 M environ par exemple, peut être utilisée sans inconvénient).

Le précipité formé de bromhydrate est filtré rapidement, lavé à l'éther et séché sous vide, sans chauffer. Sec, le sel n'est pas hygroscopique.

On obtient 16,75 g de sel (95 %).

Le produit un peu beige est décoloré par recristallisation dans environ 270 ml de benzène (avec filtration à chaud si nécessaire).

F = 134°C (Kofler).

La benzyloxy-4-pipéridine et ses sels pharmaceutiquement acceptables présentent des propriétés pharmacologiques intéressantes.En particulier

ils ont une action antispasmodique sur la musculature lisse et peuvent être utilisés en thérapeutique pour traiter les spasmes artériels (artérites, angine de poitrine), digestifs, viscéraux (coliques hépatiques et néphrétiques), vésicaux et utérins.

On donnera ci-après des résultats des études toxicologiques et pharmacologiques qui mettent en évidence ces propriétés.

a) Toxicité aigüe

La $DL_{50}$ a été déterminée chez la souris Swiss NMRI sur le bromhydrate de la benzyloxy-4 pipéridine :
- voie orale $DL_{50}$ 75 mg/kg
- voie I.V. $DL_{50}$ 32,5 mg/kg

b) Action antispasmodique

Les essais sont effectués sur le duodénum isolé de rat en survie dans du liquide de Tyrode oxygéné et à température de 37°C (cuve de 20 ml, bain-marie à organes isolés type Valette).

Le tonus et les mouvements de l'organe isolé sont enregistrés par la méthode de Magnus (R.) (Arch. Ges. Physiol. 1904, 102; 123) à l'aide d'une jauge de contrainte reliée à un enregistreur électronique Electromed.

L'antagonisme exercé par le produit étudié est recherché vis-à-vis des effets contracturants musculotropes du chlorure de baryum.

La papavérine est utilisée comme substance antagoniste de référence.

On détermine la concentration molaire du produit qui diminue de 50 % les effets du chlorure de baryum.

Les résultats sont donnés dans le tableau ci-après.

| Composé | Concentrations molaires inhibitrices 50 % |
|---|---|
| Benzyloxy-4 pipéridine (bromhydrate) | $1,8.10^{-5}$ M |
| Papavérine | $1,25.10^{-5}$ M |

Les résultats obtenus montrent que l'activité de la benzyloxy-4 pipéridine est sensiblement égale à celle de la papavérine.

La benzyloxy-4 pipéridine et de préférence ses sels pharmaceutiquement acceptables peuvent être administrés à l'homme par voie orale (notamment sous forme de gélules dosées à 10 mg 3 à 5 fois par jour) ou par voie parentérale, notamment par voie intramusculaire (sous forme de soluté aqueux à 5 mg, 2 à 3 fois par jour).

## REVENDICATIONS

1. La benzyloxy-4-pipéridine et ses sels pharmaceutiquement acceptables.

2. Procédé de préparation des composés selon la revendication 1, caractérisé en ce que

a) on condense l'hydroxy-4 pipéridine avec le chlorure de p-nitrobenzoyle en présence d'un accepteur d'acide, de façon à obtenir une pipéridine protégée de formule

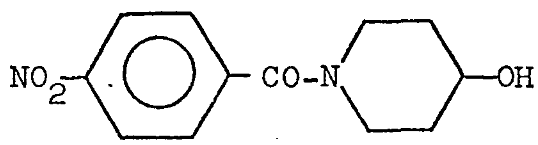

(II)

b) on effectue une benzylation du composé de formule II au moyen de chlorure de benzyle en présence d'un agent de condensation et d'un accepteur d'acide de façon à obtenir un composé de formule

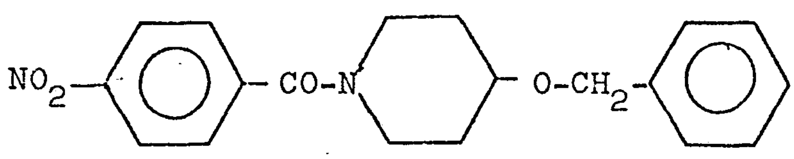

(III)

c) on libère le groupe aminé de la pipéridine, et

d) éventuellement on salifie la base ainsi obtenue.

3. Composition thérapeutique ayant une action antispasmodique, caractérisée en ce qu'elle contient à titre de principe actif un composé selon la revendication 1.

4. Composition selon la revendication 3, caractérisée en ce qu'elle contient à titre de principe actif un sel pharmaceutiquement acceptable de la benzyloxy-4-pipéridine.

5. Composition selon la revendication 3 ou la revendication 4, caractérisée en ce qu'elle est sous une forme convenant pour l'administration par voie orale.

6. Composition selon la revendication 3 ou la revendication 4, caractérisée en ce qu'elle est sous une forme convenant pour l'administration par voie parentérale.

Office européen des brevets

# RAPPORT DE RECHERCHE EUROPEENNE

0015817

Numéro de la demande

EP 80 40 0258

| DOCUMENTS CONSIDERES COMME PERTINENTS | | | CLASSEMENT DE LA DEMANDE (Int. Cl. 3) |
|---|---|---|---|
| Catégorie | Citation du document avec indication, en cas de besoin, des parties pertinentes | Revendication concernée | |
| A | FR - M - 3816 (PARKE DAVIS et CO.)<br>---- | | C 07 D 211/46<br>A 61 K 31/445 |
| | | | **DOMAINES TECHNIQUES RECHERCHES (Int. Cl. 3)** |
| | | | C 07 D 211/46<br>A 61 K 31/445 |
| | | | **CATEGORIE DES DOCUMENTS CITES**<br>X: particulièrement pertinent<br>A: arrière-plan technologique<br>O: divulgation non-écrite<br>P: document intercalaire<br>T: théorie ou principe à la base de l'invention<br>E: demande faisant interférence<br>D: document cité dans la demande<br>L: document cité pour d'autres raisons |
| X | Le présent rapport de recherche a été établi pour toutes les revendications | | &: membre de la même famille, document correspondant |

| Lieu de la recherche | Date d'achevement de la recherche | Examinateur |
|---|---|---|
| La Haye | 10-06-1980 | CREMERS |